(19) Europäisches Patentamt
European Patent Office
Office européen des brevets

(11)　**EP 4 439 565 A1**

(12)　**EUROPEAN PATENT APPLICATION**
published in accordance with Art. 153(4) EPC

(43) Date of publication:
**02.10.2024　Bulletin 2024/40**

(21) Application number: **22898906.7**

(22) Date of filing: **11.11.2022**

(51) International Patent Classification (IPC):
**G16B 20/00** (2019.01)　　**G16B 30/00** (2019.01)
**G16B 40/00** (2019.01)

(52) Cooperative Patent Classification (CPC):
**G16B 20/00; G16B 30/00; G16B 40/00**

(86) International application number:
**PCT/KR2022/017675**

(87) International publication number:
**WO 2023/096224 (01.06.2023 Gazette 2023/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.11.2021　KR 20210163842**

(71) Applicant: **Genomecare Co., Ltd.**
**Suwon-si, Gyeonggi-do 16229 (KR)**

(72) Inventors:
• **KIM, Sunshin**
**Yongin-si Gyeonggi-do 17003 (KR)**
• **PRASAD, Adhikari Krishna**
**Hwaseong-si Gyeonggi-do 18261 (KR)**
• **JEONG, Jae Hwan**
**Hanam-si Gyeonggi-do 12916 (KR)**
• **OH, Gyeong In**
**Seoul 07051 (KR)**

(74) Representative: **HGF**
**HGF Limited**
**1 City Walk**
**Leeds LS11 9DX (GB)**

(54)　**METHOD FOR DETECTING CHROMOSOME ANEUPLOIDY OF FETUS ON BASIS OF VIRTUAL DATA**

(57)　Provided are: a method for detecting chromosome aneuploidy of a fetus on the basis of virtual data; and a computer-readable medium for recording a program applied to perform the method. According to the present invention, non-invasive prenatal diagnosis of chromosome aneuploidy in a fetus can be performed with excellent sensitivity and specificity.

Figure 1d

T13 actual data

Sensitivity = 99.47%

Positive predictive value = 99.77%

EP 4 439 565 A1

## Description

### Technical Field

There is provided a method for detecting chromosome aneuploidy of a fetus on the basis of virtual data, i.e., virtual chromosome aneuploidy data.

### Background Art

Prenatal diagnosis refers to diagnosing the presence or absence of a disease in the fetus before the fetus is born. Prenatal diagnosis is largely divided into invasive and non-invasive diagnostic methods. Invasive diagnostic methods include, for example, chorionic screen, amniotic fluid test, and umbilical cord puncture. Invasive diagnostic methods have the potential to cause miscarriage, disease, or deformity by causing shock to the fetus during the test process, and thus non-invasive diagnostic methods that are performed prior to the invasive methods are being developed.

Recently, it has been demonstrated that non-invasive diagnosis of fetal chromosome aneuploidy is feasible by massive parallel sequencing of DNA molecules in the plasma of pregnant women. Fetal DNA can be detected in maternal plasma and serum from the 7th week of pregnancy, and the amount of fetal DNA in maternal blood increases with the period of pregnancy. When performing massive parallel sequencing of fetal DNA, the threshold for distinguishing a normal fetus from a chromosome aneuploidy fetus is unclear, resulting in low sensitivity and specificity for chromosome aneuploidy detection.

Therefore, there is a need to develop a method that can clearly distinguish a normal fetus from a chromosome aneuploidy fetus and increase the sensitivity and specificity of chromosome aneuploidy detection.

### Prior Art Document

### Patent Document

(Patent Document 1) KR 1739535 B

### Detailed Description of Invention

### Technical Problem

There is provided a method for detecting chromosome aneuploidy of a fetus.

There is provided a computer-readable medium for recording a program applied to perform the method.

### Solution to Problem

In one aspect, there is provided a method for detecting chromosome aneuploidy of a fetus, comprising:

obtaining sequence information (read) of a plurality of nucleic acid fragments obtained from a biological sample of a pregnant woman with a normal fetus;

mapping the obtained sequence information to a human reference genome and aligning the sequence information of the nucleic acid fragments to a chromosome;

calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;

producing virtual positive data from normal data based on the read count and fetal DNA fraction in the target chromosome in a normal fetus;

establishing a chromosome aneuploidy discrimination model from the produced virtual positive data and normal data; and

detecting chromosome aneuploidy of a test sample using the chromosome aneuploidy discrimination model.

The term "chromosome aneuploidy" refers to a condition in which the number of chromosomes per cell in a cell, individual, or lineage is not an integer multiple of the base number, and is one to several more or less than an integer multiple, that is, a condition involving a genome with an incomplete structure. In the case of diploidy, a case in which both two of a pair of homologous chromosomes are missing is referred to as nullisomy, and a case in which one of a pair of homologous chromosomes is missing and only the other is present is referred to as monosomy, and a case in which one extra chromosome is present in addition to one pair of homologous chromosomes is referred to as trisomy (T).

The chromosome aneuploidy may be trisomy 13, trisomy 18, trisomy 21, XO, XXX, XXY, XYY, or a combination thereof. Abnormalities of chromosome 13 (trisomy 13) are associated with Patau syndrome. Abnormalities of chromosome 18 (trisomy 18) are associated with Edwards syndrome. Abnormalities of chromosome 21 (trisomy 21) are associated with Down syndrome. Monosomy X (XO, i.e., the absence of one X chromosome) is associated with Turner syndrome. XXY is a condition in which human males have an extra X chromosome and is associated with Klinefelter syndrome.

The method comprises obtaining sequence information (read) of a plurality of nucleic acid fragments obtained from a biological sample of a pregnant woman with a normal fetus.

The pregnant woman may be a pregnant woman with a single fetus or twin fetuses.

The biological sample may be blood, plasma, serum, urine, saliva, mucosal secretions, sputum, feces, tears, or a combination thereof. The biological sample is, for example, peripheral blood plasma. The biological sample may include a nucleic acid of a fetus. The nucleic acid of a fetus may be cell-free DNA (cfDNA). The nucleic acid of a fetus may be isolated DNA.

The obtaining sequence information of a plurality of nucleic acid fragments obtained from a biological sample of a pregnant woman may comprise isolating nucleic acids from a biological sample.

The method of isolating nucleic acids from a biological sample may be performed by methods known to those of ordinary skill in the art. A length of the isolated nucleic acid fragment may be about 10 bp (base pair) to about 2000 bp, about 15 bp to about 1500 bp, about 20 bp to about 1000 bp, about 20 bp to about 500 bp, about 20 bp to about 200 bp, or about 20 bp to about 100 bp.

The obtaining sequence information of a plurality of nucleic acid fragments from a biological sample obtained from a pregnant woman may include performing massive parallel sequencing on the isolated nucleic acids.

The term "massive parallel sequencing" may be used interchangeably with next-generation sequencing (NGS) or second-generation sequencing. Massive parallel sequencing refers to a technique for simultaneously sequencing millions of fragments of nucleic acids. Massive parallel sequencing may be performed in parallel, for example, by 454 Platform (Roche), GS FLX Titanium, Illumina MiSeq, Illumina HiSeq, Illumina Genome Analyzer, Solexa platform, SOLiD System (Applied Biosystems), Ion Proton (Life Technologies), Complete Genomics, Helicos Biosciences Heliscope, single molecule real-time (SMRT™) technology from Pacific Biosciences, or a combination thereof.

The method may further comprise preparing a nucleic acid library in order to perform massive parallel sequencing.

The nucleic acid library may be prepared according to the method of massive parallel sequencing. The nucleic acid library may be constructed according to the manufacturer's instructions that provide massive parallel sequencing.

The sequence information of the obtained nucleic acid fragment may also be referred to as a read.

The method comprises mapping the obtained sequence information to a human reference genome and aligning the sequence information of the nucleic acid fragments to a chromosome.

The human reference genome may be hg18 or hg19. The sequence information that is mapped to only one genomic location in a human reference genome may be aligned as unique sequence information. Based on the aligned unique sequence number, the sequence information of the nucleic acid fragment may be aligned to the location of the chromosome. The location of the chromosome may be a contiguous range on a chromosome having a length of at least about 5 kb, about 10 kb, about 20 kb, about 50 kb, about 100 kb, about 1000 kb, or 2000 kb. The location of the chromosome may be a single chromosome.

After aligning the sequence information of the nucleic acid fragment to a chromosome, the method may further comprise checking the depth distribution of the sequence information of the nucleic acid fragments aligned to the chromosome for each section and excluding sections with low reliability of the sequence information from the analysis target. The section may be a section set in a unit of about 5 kb to about 50 kb. For example, the section may be a section set to about 10 kb to about 40 kb, about 15 kb to about 30 kb, or about 20 kb to about 25 kb. By setting the above section, filtering may be done using the GC content of the nucleotide sequence. In addition, by setting the above section, a group of the depth and GC content of the sequence information of the nucleic acid fragments aligned to the chromosome may be form, and statistical analysis may be performed.

The excluding sections with low reliability of the sequence information from the analysis target may comprise removing mismatch portions, removing the sequence information aligned to multiple sites, removing duplicated sequence information, or a combination thereof. In order to exclude sections with low reliability of the sequence information from the analysis target, quality filtering, trimming, perfect match, removal of sequences aligned to multiple locations, removal of PCR duplicated sequence information (PCR duplicated reads), or a combination thereof may be performed. The quality filtering is a process of extracting sequence information with high quality for the quality of each nucleotide sequence obtained during the sequencing process. The trimming is a process of removing parts of poor quality because the quality of the latter part of the nucleotide sequence is low due to the nature of the sequencing device. For example, nucleic acid fragments may be trimmed to a size of at least about 50 bp, greater than about 50 bp, or greater than about 100 bp. For example, the quality value of the nucleic acid fragment may be 20 or higher, 30 or higher, 40 or higher, or 50 or higher. The perfect match selects only nucleotide sequences that perfectly match when mapping to a human reference genome. Since sequences aligned to multiple locations are likely to be repetitive sequence regions, sequences aligned to multiple locations may be removed from the obtained sequence information. Removing the PCR duplicated sequence information means removing parts that have been amplified more due to errors during the sequencing process. In addition, for statistical analysis, a group with a certain degree of even deviation must be selected to obtain meaningful results. The part without depth is usually the N-region of the chromosome and may be excluded from the analysis target.

In the above method, based on the sequence information of the nucleic acid fragments aligned to the chromosome, the total number assigned to chromosome 1 may be denoted as C1RC (chromosome 1 read count), and the total number assigned to chromosome 2 may be denoted as C2RC (chromosome 2 read count). Therefore, the total number assigned to chromosome i may be denoted as CiRC (chromosome i read count). In addition, in the case of a sex chromosome, the total number assigned to X chromosome may be denoted as CXRC (chromosome X read count), and the total number assigned to Y chromosome may be denoted as CYRC (chromosome Y read count). The total number assigned to autosome 1 to autosome 22 is denoted as ATRC (autosomal total read count), and the total number assigned to all autosomes and sex chromosomes is denoted as TRC (total read count).

The method comprises calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome.

The target chromosome may be selected from the group consisting of chromosome 1 to chromosome 22, X chromosome, and Y chromosome. The target chromosome may be selected from the group consisting of chromosome 13, chromosome 18, and chromosome 21.

The term "read count (RC)" or "read number" is a value that counts the number of reads aligned to the location of one gene.

The term "fetal DNA fraction (fetal fraction: FF)" or "fraction of fetal nucleic acids" refers to the amount or ratio (%) of fetal nucleic acids among nucleic acids isolated from a biological sample of a pregnant woman. The fetal fraction may be a concentration, relative ratio, or absolute amount of fetal nucleic acids. The fetal nucleic acid may be a nucleic acid derived from fetal placenta trophoblasts.

The term "GC content" refers to the ratio (%) of guanine (G) and cytosine (C) among the bases that make up DNA. The GC content may be calculated from the equation of GC content = $(G+C)/(A+T+G+C)$.

The method comprises producing virtual positive data from normal data based on the read count and fetal DNA fraction in the target chromosome in a normal fetus.

The normal data may be the read count in the target chromosome in a normal fetus.

The positive data is the read count in the target chromosome in a fetus with chromosome aneuploidy, and may be the virtual read count obtained from the normal data.

The producing virtual positive data may use CiRC (chromosome i read count) (i = 1 to 22) and FF in order to detect autosome aneuploidy. For example, in the case of chromosome 13, C13RC and FF are used.

The producing virtual positive data from normal data may be determined by the equation $TiRC = CiRC + CiRC \times FF/2$ when the target chromosome is an autosome. In the above equation, TiRC is the read count in the target chromosome i in a chromosome aneuploidy fetus, CiRC is the read count in the target chromosome i in a normal fetus, and FF is the fetal DNA fraction.

The producing virtual positive data from normal data may be determined by the equation $CXRC = CXXRC - CXXRC \times FF/2$ when the target chromosome is XO chromosome. In the above equation, CXRC is the read count in the target chromosome XO in a chromosome aneuploidy fetus, CXXRC is the read count in the target chromosome XX in a normal female fetus, and FF is the fetal DNA fraction.

The producing virtual positive data from normal data may be determined by the equation $CXXXRC = CXXRC + CXXRC \times FF/2$ when the target chromosome is XXX chromosome. In the above equation, CXXXRC is the read count in the target chromosome XXX in a chromosome aneuploidy fetus, CXXRC is the read count in the target chromosome XX in a normal female fetus, and FF is the fetal DNA fraction.

The producing virtual positive data from normal data may be determined by the equation $CXYYRC = CXYRC + CYRC$ when the target chromosome is XYY chromosome. In the above equation, CXYYRC is the read count in the target chromosome XYY in a chromosome aneuploidy fetus, CXYRC is the read count in the target chromosome XY in a normal male fetus, and CYRC is the read count assigned to Y chromosome in a normal male fetus.

The producing virtual positive data from normal data may be determined by the equation $CXXYRC = CXXRC + CYRC$ when the target chromosome is XXY chromosome. In the above equation, CXXYRC is the read count in the target chromosome XXY in a chromosome aneuploidy fetus, CXXRC is the read count in the target chromosome XX in a normal female fetus, and CYRC is the read count assigned to Y chromosome. The CYRC may be calculated by a method comprising calculating a residual from

$$\mathrm{CYRC} = \beta_0 + \beta_1 TRC + \beta_2 FF_{chrY} + \varepsilon \quad \text{(equation 1)}$$

and

$$CYRC' = \beta_0 + \beta_1 TRC + \beta_2 FF_{chrY} \quad \text{(equation 2)}.$$

In the above equation 1, TRC is the read count in the entire chromosome, $FF_{chrY}$ is FF based on the Y chromosome, $\beta_0$ is an intercept, $\beta_1$ is the coefficient between TRC and CYRC, $\beta_2$ is the coefficient between $FF_{chrY}$ and CYRC, and $\varepsilon$ is a residual. In the above equation 2, CYRC' is the predicted value of CYRC, TRC is the read count in the entire chromosome, $FF_{chrY}$ is FF based on the Y chromosome, $\beta_0$ is an intercept, $\beta_1$ is the coefficient between TRC and CYRC', and $\beta_2$ is the coefficient between $FF_{chrY}$ and CYRC'. The method may comprise calculating CYRC from the calculated residual. Equations 1 and 2 may be modeled using male fetus (XY) samples. The distribution of the residuals may be assumed to follow a normal distribution and may have a standard deviation indicating the degree of spread of the normal distribution. In addition, for samples with XXY chromosome aneuploidy, a regression model similar to that of male fetus samples and a normal distribution of residuals according to this model may be assumed. Since the read count assigned to Y chromosome (CYRC) is relatively small and negligible compared to the TRC, the TRC and FF (machine learning-based) of the female fetus (XX) sample may be considered to be the TRC and FF of the sample with XXY chromosome aneuploidy. By calculating CYRC' from equation 2, and using equation 1 to randomly generate residuals according to a normal distribution with the same standard deviation as the residual distribution of a male fetus sample described above and adding them to CYRC', the CYRC of a female fetuse may be approximately obtained.

The method comprises establishing a chromosome aneuploidy discrimination model from the produced virtual positive data and normal data.

The establishing a chromosome aneuploidy discrimination model from the produced virtual positive data and normal data may be performed based on CiRC (chromosome i read count) (i = 1 to 22), ATRC (autosomal total read count), FF, and GC content when the target chromosome is an autosome.

The establishing a chromosome aneuploidy discrimination model from the produced virtual positive data and normal data may be performed based on CXRC (chromosome X read count), CYRC (chromosome Y read count), or a combination thereof; TRC (total read count); FF; and GC content when the target chromosome is a sex chromosome.

The chromosome aneuploidy discrimination model may be trained by applying a machine learning algorithm. The machine learning algorithm may be selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis). The LGBM may be described in Guolin Ke et al., "LightGBM: A Highly Efficient Gradient Boosting Decision Tree". Proceedings of the 31st International Conference on Neural Information Processing Systems (NIPS 2017), pp. 3149-3157.

The method comprises detecting chromosome aneuploidy of a test sample using the chromosome aneuploidy discrimination model.

The detecting chromosome aneuploidy of a test sample using the chromosome aneuploidy discrimination model may be performed based on CiRC (i = 1 to 22), ATRC, FF, and GC content when the target chromosome is an autosome.

The detecting chromosome aneuploidy of a test sample using the chromosome aneuploidy discrimination model may be performed based on CXRC, CYRC, or a combination thereof; TRC, FF, and GC content when the target chromosome is a sex chromosome.

In another aspect, there is provided a computer-readable medium for recording a program applied to perform the method according to one aspect. The computer-readable medium encompasses a system that comprises a computer-readable medium.

**Effects of Invention**

According to a method for detecting chromosome aneuploidy of a fetus on the basis of virtual data according to an embodiment, and a computer-readable medium for recording a program applied to perform the method, non-invasive prenatal diagnosis of chromosome aneuploidy in a fetus can be performed with excellent sensitivity and specificity.

**Brief Description of Drawings**

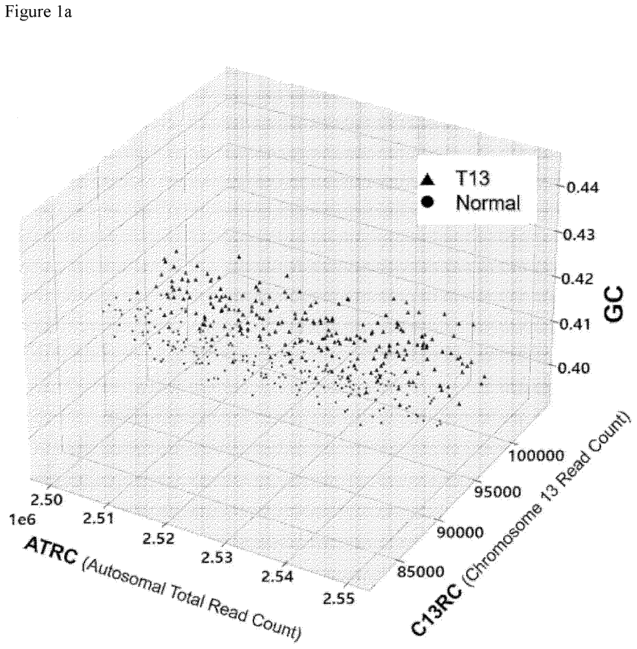

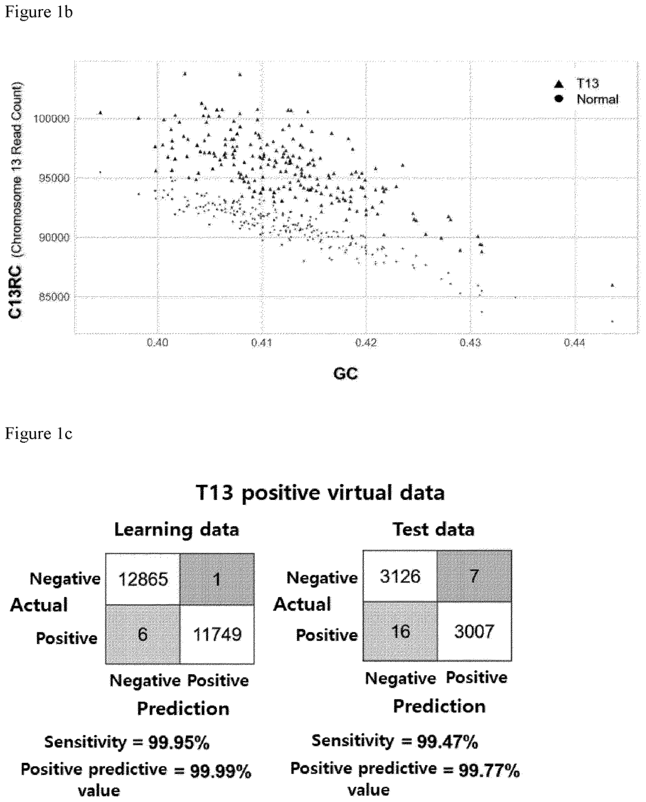

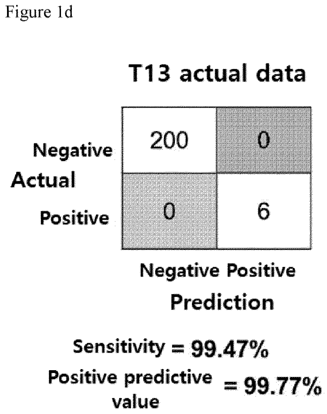

Figure 1a shows the distribution of positive and negative data according to three axes (ATRC, GC content, and C13RC) targeting chromosome 13 in three dimensions, Figure 1b shows the distribution of positive and negative data according to GC content and C13RC in a plane, Figure 1c shows the results showing the sensitivity and positive predictive value of a test sample that was learned using 80% of the data (learning data) and verified using 20% of the data (test data) after randomly mixing data in a model using virtual data, and Figure 1d shows the results obtained by further verifying the accuracy of the established model using a positive sample definitely diagnosed by amniotic fluid test.

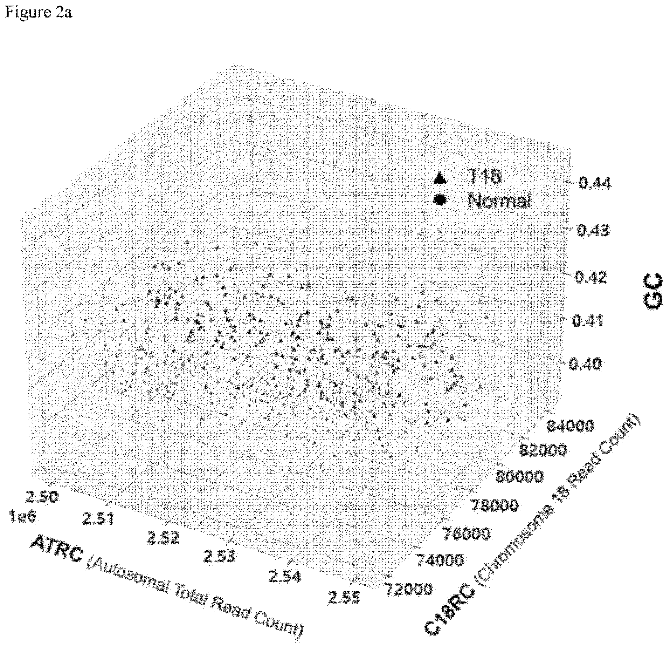

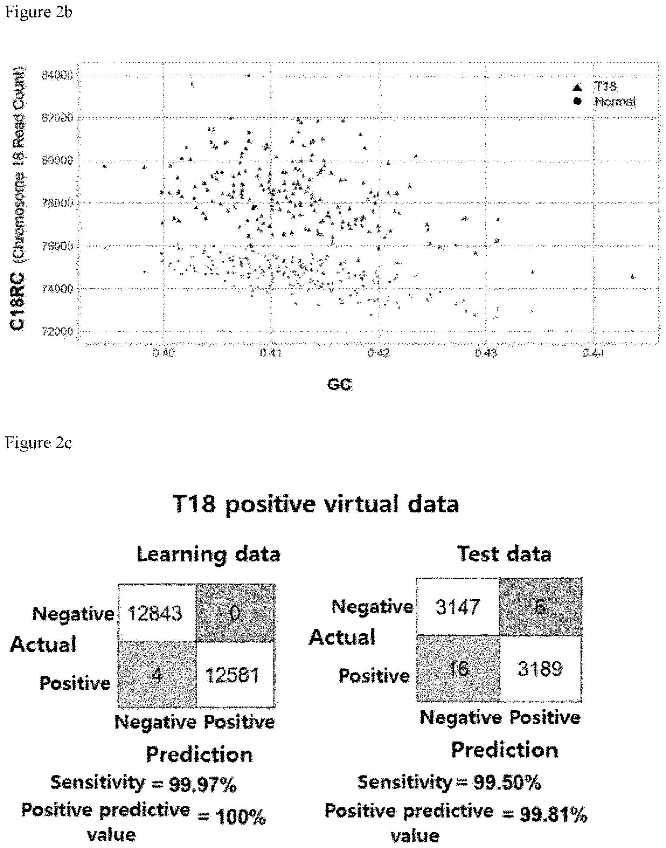

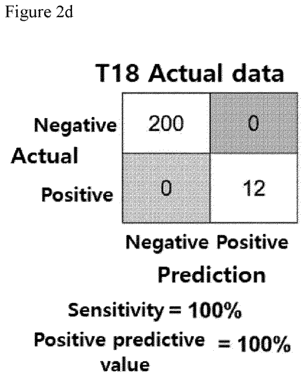

Figure 2a shows the distribution of positive and negative data according to three axes (ATRC, GC and C18RC) targeting chromosome 18 in three dimensions, Figure 2b shows the distribution of positive and negative data according to GC content and C18RC in a plane, Figure 2c shows the results showing the accuracy of a test sample that was learned using 80% of the data (learning data) and verified using 20% of the data (test data) after randomly mixing data in a model using virtual data, and Figure 2d shows the results obtained by further verifying the accuracy of the established model using a positive sample definitely diagnosed by amniotic fluid test.

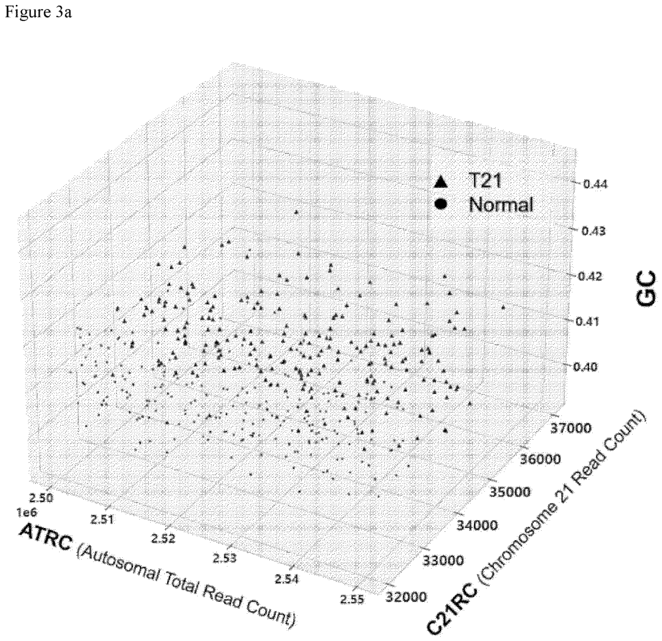

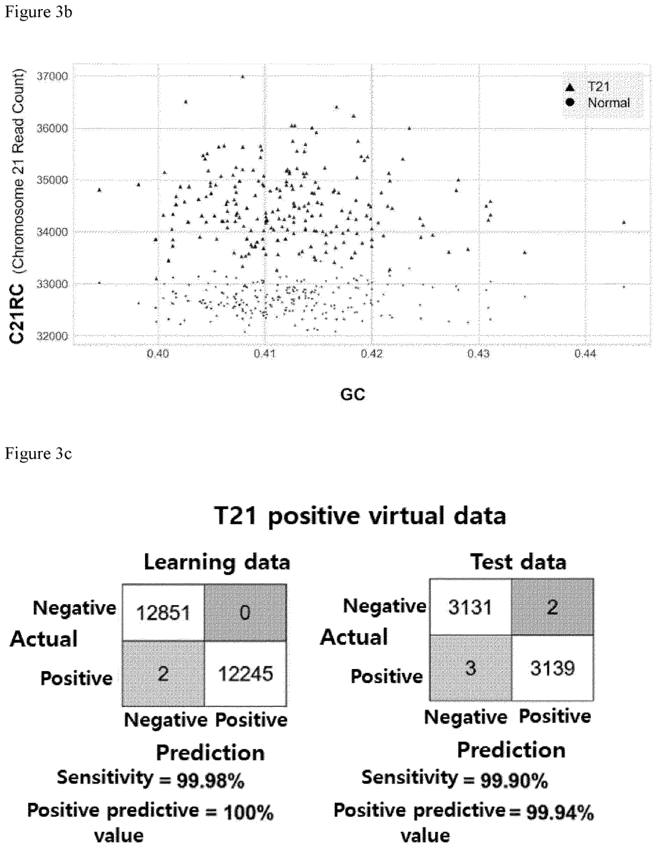

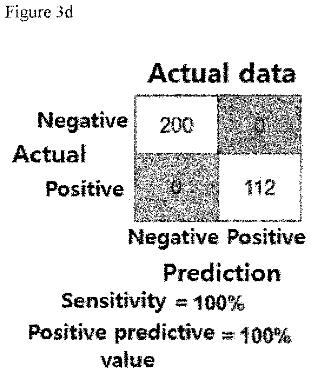

Figure 3a shows the distribution of positive and negative data according to three axes (ATRC, GC content, and C21RC) targeting chromosome 21 in three dimensions, Figure 3b shows the distribution of positive and negative data according to GC content and C21RC in a plane, Figure 3c shows the results showing the accuracy of a test sample that was learned using 80% of the data (learning data) and verified using 20% of the data (test data) after randomly mixing data in a model using virtual data, and Figure 3d shows the results obtained by further verifying the accuracy of the established model using a positive sample definitely diagnosed by amniotic fluid test.

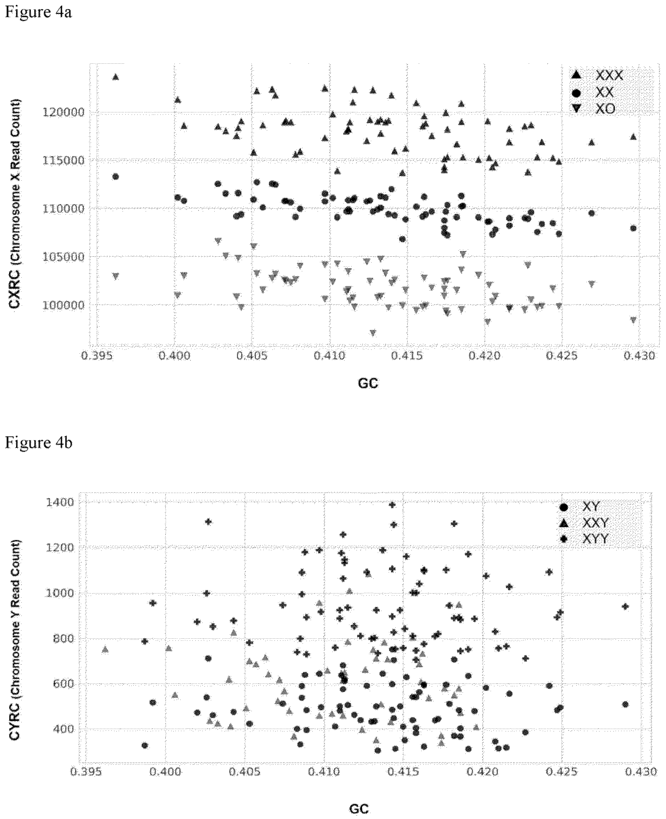

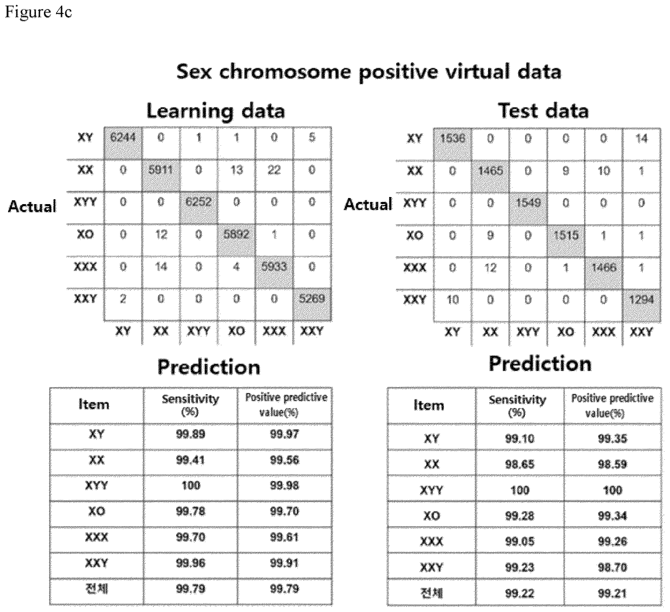

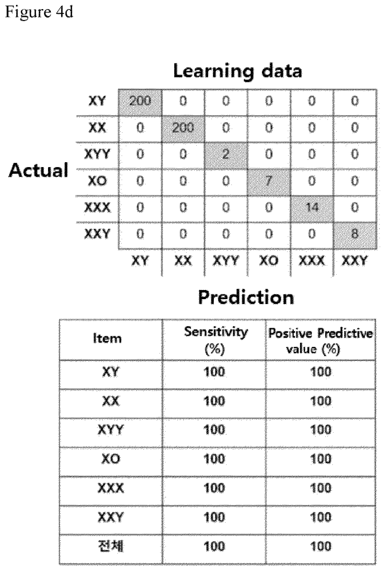

Figure 4a shows the distribution of samples of a normal female fetus (XX), a X monosomy (XO), and a triple X syndrome (XXX) according to two axes (GC content and CXRC) targeting the female fetus XX chromosome, Figure 4b shows the distribution of data of a normal male fetus (XY), Klinefelter syndrome (XXY), and XYY syndrome (XYY) according to two axes (GC and CYRC) targeting the male fetus XY chromosome, Figure 4c shows the results showing the accuracy of a test sample that was learned using 80% of the data (learning data) and verified using 20% of the data (test data) after randomly mixing data in a model using positive virtual data, and Figure 4d shows the results obtained by further verifying the accuracy of the established model using a positive sample definitely diagnosed by amniotic fluid test.

**Best Mode for Carrying out the Invention**

Hereinafter, the present invention will be described in more detail through examples. However, these examples are intended to illustrate the present invention, and the scope of the present invention is not limited to these examples.

**Example 1. Non-invasive detection of fetal chromosome aneuploidy based on virtual data production**

**1. Preparation of samples**

Blood from a total of 15,999 pregnant women with a fetus was collected. Among these, amniotic fluid tests for karyotyping of the fetus were performed on those subjects who were detected as positive.

All subjects underwent standard prenatal chromosome aneuploidy screening at a accredited clinical trial center. First trimester screening includes measurements of serum pregnancy-associated plasma protein A (PAPP-A), total or free beta subunit of human chorionic gonadotropin (hCG), and fetal nuchal translucency. Second trimester screening includes measurements of maternal serum alpha-fetoprotein (MSAFP), hCG, unconjugated estriol, and inhibin A.

As a result of karyotype analysis, 6 fetuses had aneuploidy on chromosome 13, 12 fetuses had aneuploidy on chromosome 18, 112 fetuses had aneuploidy on chromosome 21, 7 fetuses had an XO chromosome, 8 fetuses had an XXY chromosome, 2 fetuses had an XYY chromosome, and 14 fetuses had an XXX chromosome, indicating that chromosome aneuploidy was definitely diagnosed in the actual data.

**2. Preparation of cell-free DNA and DNA library for DNA sequencing**

Approximately 10 ml of peripheral blood was drawn from the subjects as described in 1. and collected in a BCT™ tube (Streck, Omaha, NE, USA). The collected blood samples were centrifuged at 1,200 × g at 4 °C for 15 minutes. Blood plasma was collected and centrifuged again at 16,000 × g at 4 °C for 10 minutes. Cell-free DNA (cfDNA) was obtained from the centrifuged plasma using the MagListo™ cfDNA extraction kit (Bioneer, Korea).

The obtained cfDNA fragment was end-repaired using T4 DNA polymerase, Klenow DNA polymerase, and T4 polynucleotide kinase, and the cfDNA fragment was obtained again using the Agencourt AMPure XP.

A DNA library for the ion proton sequencing system was constructed from the prepared cfDNA according to the protocol provided by the manufacturer (ThermoFisher Scientific). Using the 540 chip, an average 0.3x sequencing coverage depth per nucleotide was calculated.

### 3. Massive parallel sequencing

The DNA library prepared as described in 2. was subjected to massive parallel sequencing using the Ion Torrent S5XL™ system (ThermoFisher Scientific).

Different raw reads were obtained using Ion Torrent Suite™ software (ThermoFisher Scientific). The filtered reads were aligned to the human genome reference sequence hg19 by Burrows-Wheeler transform (BWT). Sequence reads that were mapped to only one genomic location in hg19 were aligned as unique reads. Of the total reads, approximately $3.3 \times 10^6$ were unique reads, and the GC content of the total 15,999 samples ranged from approximately 39% to 45%.

### 4. Production and detection of virtual positive data with chromosome aneuploidy from normal data

### A. Method of producing virtual positive data with trisomy from normal autosomal data

A method of producing a trisomy from an autosome is to add the read count (RC) of autosome i plus the result of multiplying the read count (RC) of autosome i by 50% of the fetal DNA fraction (fetal fraction: FF).

A specific method of producing chromosome aneuploidy by targeting an autosome is explained using chromosomes 13, 18, and 21 as examples.

When any one of samples is selected and chromosome 13 is targeted, the DNA reads of chromosome 13 are mixed with maternal and fetal reads. At this time, it is important to know the ratio of the DNA reads in a fetus as accurately as possible. Therefore, in the case of a male fetus, a method based on the Y chromosome is used, and in the case of a female fetus, a method using FF based on machine learning is used.

In the case of a normal fetus, the number of DNA reads assigned to chromosome 13 is determined by deriving from a pair of maternal chromosomes and a pair of fetal chromosomes. Therefore, the chromosomal DNA read count in a normal fetus is determined by multiplying the total DNA read count assigned to chromosome 13 by the fetal DNA fraction (FF). On the other hand, because trisomy 13 has one additional fetal chromosome, an additional 50% must be added to the normal fetal DNA read count. As a result, if the DNA read count assigned to chromosome 13 in any sample is C13RC, chromosome 13 T13RC of the trisomy sample can be calculated by C13RC + C13RC * FF/2. As such, T18RC and T21RC can be obtained by applying this method to chromosomes 18 and 21.

### B. Detection of positive data with trisomy in autosome

### (1) Detection of trisomy chromosome 13

When targeting chromosome 13, 14,778 virtual data with trisomy were produced from a total of 15,999 normal data.

The results of displaying the distribution of positive and negative data according to three axes (ATRC, GC content, and C13RC) targeting chromosome 13 in three dimensions are shown in Figure 1a, and the results of displaying the distribution of positive and negative data according to GC content and C13RC in a plane are shown in Figure 1b. Figure 1a shows the distribution between the total read count of DNA reads assigned to autosomes on chromosome 13 (autosomal total read count: ATRC), and the total read count of DNA reads assigned to chromosome 13 (C13RC), and GC content for trisomy chromosome 13 (T13) and normal chromosome 13 (Normal). From these results, ATRC, C13RC, and GC content are expected to serve as parameters that can classify T13 and normal samples. In particular, Figure 1b shows that C13RC according to changes in GC content within the limited range of ATRC (2,450,000 to 2,500,000) can be clearly distinguish between trisomy data and normal data.

A model was established using the LGBM algorithm for 15,999 negative data and 14,778 positive virtual data using three parameters: ATRC, C13RC, and GC content. The results showing the sensitivity and positive predictive value of a test sample that was learned using 80% of the data (learning data) and verified using 20% of the data (test data) after randomly mixing data are shown in Figure 1c. As shown in Figure 1c, it was confirmed that the sensitivity and positive predictive value were each at least 99% in the learning and test data.

In addition, the results obtained by further verifying the accuracy of the established model using a positive sample definitely diagnosed by amniotic fluid test are shown in Figure 1d. As shown in Figure 1d, when this model using virtual data was used, it was accurately predicted that the six actual positive samples (T13) confirmed by amniotic fluid test were positive data.

**(2) Detection of trisomy chromosome 18**

When targeting chromosome 18, 15,790 virtual data with trisomy were produced from a total of 15,996 normal data. Data was selected and presented within the ATRC range (2,450,000 to 2,500,000). Some data that were statistically outside the normal range were removed depending on the characteristics of each target chromosome.

The results of displaying the distribution of positive and negative data according to three axes (ATRC, GC and C18RC) targeting chromosome 18 in three dimensions are shown in Figure 2a, and the results of displaying the distribution of positive and negative data according to GC content and C18RC in a plane are shown in Figure 2b. The results showing the accuracy of a test sample that was learned using 80% of the data (learning data) and verified using 20% of the data (test data) after randomly mixing data in a model using virtual data are shown in Figure 2c, and the results obtained by further verifying the accuracy of the established model using a positive sample definitely diagnosed by amniotic fluid test are shown in Figure 2d.

Figures 2a and 2b also showed that ATRC, C18RC and GC content can classify trisomy chromosome 18 (T18) and normal chromosome (Normal). In Figure 2c, it was confirmed that the sensitivity and positive predictive value were each at least 99% in the learning and test data. In Figure 2d, when the method using virtual data was used, it was accurately predicted that the 12 actual positive samples (T18) confirmed by amniotic fluid test were positive data.

**(3) Detection of trisomy chromosome 21**

When targeting chromosome 21, 15,389 virtual data with trisomy were produced from a total of 15,984 normal data. Data was selected and presented within the ATRC range (2,450,000 to 2,500,000). Some data that were statistically outside the normal range were removed depending on the characteristics of each target chromosome.

The results of displaying the distribution of positive and negative data according to three axes (ATRC, GC and C21RC) targeting chromosome 21 in three dimensions are shown in Figure 3a, and the results of displaying the distribution of positive and negative data according to GC content and C21RC in a plane are shown in Figure 3b. The results showing the accuracy of a test sample that was learned using 80% of the data (learning data) and verified using 20% of the data (test data) after randomly mixing data in a model using virtual data are shown in Figure 3c, and the results obtained by further verifying the accuracy of the established model using a positive sample definitely diagnosed by amniotic fluid test are shown in Figure 3d.

In addition, in Figures 3a and 3b, the difference between trisomy chromosome 21 (T21) and normal chromosome (Normal) was confirmed. In Figure 3c, the sensitivity and positive predictive value were at least 99% in the learning and test data. In Figure 3d, when the method using virtual data was used, it was accurately predicted that the 112 actual positive samples (T21) confirmed by amniotic fluid test were positive data.

**C. Method of producing virtual positive data with sex chromosome aneuploidy from normal sex chromosome data**

In order to produce data with a sex chromosome aneuploidy in a fetus, male fetus (XY) data, female fetus (XX) data, and fetal DNA fraction (FF) were used.

For FF, Y chromosome-based FF was used for male fetus samples, and machine learning-based FF was used for female fetus samples.

The method of producing virtual data representing XO sex chromosome aneuploidy is to subtract the read count (CXXRC) multiplied by 50% of FF from the read count (chromosome XX read count: CXXRC) in a female fetus. In other words, the read count of a fetus with XO chromosome aneuploidy (chromosome XO read count: CXORC) can be calculated by CXXRC - CXXRC * FF/2.

The method of producing the read count of a fetus with XXX sex chromosome aneuploidy is to add the read count in any female fetus samples (CXXRC) plus the read count (CXXRC) multiplied by 50% of FF. In other words, CXXXRC can be calculated by CXXRC + CXXRC * FF/2.

The method of producing the read count of a fetus with XYY sex chromosome aneuploidy is to add the read count in any male fetus samples (chromosome XY read count: CXYRC) plus the read count assigned to Y chromosome in the male fetus samples (chromosome Y read count: CYRC). In other words, the read count of a fetus with XYY chromosome aneuploidy (chromosome XYY read count: CXYYRC) can be calculated by CXYRC + CYRC.

The method of producing the read count of a fetus with XXY sex chromosome aneuploidy (chromosome XXY read count: CXXYRC) is as follows. First, using 7,801 male fetus samples using Y chromosome-based FF,

$$\text{CYRC} = \beta_0 + \beta_1 TRC + \beta_2 FF_{chrY} + \varepsilon \quad \text{(equation 1)}$$

is expressed as the following regression equation (CYRC: chromosome Y read count, $\beta_0$: intercept, $\beta_1$: the coefficient between TRC and CYRC, TRC: total read count, $\beta_2$: the coefficient between fetal DNA fraction of Y chromosome and CYRC, FF: fetal DNA fraction (fetal fraction), chrY: chromosome Y, and $\varepsilon$: residual). As such, TRC and chromosome Y-based FF were used as independent variables. The predicted value of the dependent variable can be expressed as

$$CYRC' = -587 + 0.0002 \times TRC + 62 \times FF_{chrY} \quad \text{(equation 2)}$$

(CYRC': chromosome Y read count predicted by regression analysis).

From the above two equations, the standard deviation of the residual was calculated as 44.5, and a new residual was calculated by normal distribution according to this standard deviation. CYRC' was calculated from equation 2 using the total read count (TRC) in any female fetuses and machine learning-based FF, and the new residual obtained previously was added to calculate the new CYRC. As a result, CXXYRC can be obtained by adding the newly obtained CYRC to the CXXRC in any female fetus samples.

**D. Detection of positive data with sex chromosome aneuploidy on sex chromosomes**

A graph representing the distribution of samples of a normal female fetus (XX), a X monosomy (XO), and a triple X syndrome (XXX) according to two axes (GC and CXRC) targeting the female fetus XX chromosome within the range (2,300,000 to 2,350,000) of the total read count (TRC) in two dimensions in a plane is shown in Figure 4a. As shown in Figure 4a, there was a difference between the distributions of CXXRC, CXORC, and CXXXRC in the read count assigned to the X chromosome (CXRC) according to GC content in the target female fetus X chromosome (XX).

A graph representing the distribution of data of a normal male fetus (XY), Klinefelter syndrome (XXY), and XYY syndrome (XYY) according to two axes (GC content and CYRC) targeting the male fetus XY chromosome within the range (2,450,000 to 2,500,000) of the TRC in a plane is shown in Figure 4b. As shown in Figure 4b, it was confirmed that there was a difference between the distributions of CXYRC, CXXYRC, and CXYYRC in the read count assigned to the Y chromosome (CYRC) according to GC content in the target male fetus Y chromosome (XY).

Due to these differences in distribution, CXRC, CYRC, and GC content can be used as parameters to classify a normal fetus sample such as XX and XY and a sex chromosome aneuploidy sample with XO, XXX, XXY, or XYY.

In fact, 7,431 normal female fetuses and 7,801 normal male fetuses were used to produce 7801 XYY, 7431 XO, 7431 XXX, and 6575 XXY positive virtual data, respectively, and a model was established using the LGBM algorithm. The results showing the accuracy of a test sample that was learned using 80% of the data and verified using 20% of the data after randomly mixing data are shown in Figure 4c. As shown in Figure 4c, it was confirmed that the learning and test data showed an accuracy of about 99% in sensitivity and positive predictive value, respectively.

The results obtained by further verifying the accuracy of the established model using a positive sample definitely diagnosed by amniotic fluid test are shown in Figure 4d. As shown in Figure 4d, the actual positive samples of 2 XYY, 7 XO, 14 XXX, and 8 XXY confirmed by amniotic fluid test were predicted with 100% accuracy.

**Claims**

1. A method for detecting chromosome aneuploidy of a fetus, comprising:

   obtaining sequence information (read) of a plurality of nucleic acid fragments obtained from a biological sample of a pregnant woman with a normal fetus;
   mapping the obtained sequence information to a human reference genome and aligning the sequence information of the nucleic acid fragments to a chromosome;
   calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;
   producing virtual positive data from normal data based on the read count and fetal DNA fraction in the target chromosome in a normal fetus, wherein the normal data is the read count in the target chromosome in a normal fetus, and the positive data is the read count in the target chromosome in a fetus with chromosome aneuploidy;
   establishing a chromosome aneuploidy discrimination model from the produced virtual positive data and normal data; and
   detecting chromosome aneuploidy of a test sample using the chromosome aneuploidy discrimination model.

2. The method according to claim 1, wherein the target chromosome is selected from the group consisting of chromosome 1 to chromosome 22, X chromosome, and Y chromosome.

3. The method according to claim 2, wherein the target chromosome is selected from the group consisting of chromosome 13, chromosome 18, and chromosome 21.

4. The method according to claim 1, wherein producing virtual positive data from normal data is determined by the following equation when the target chromosome is an autosome:

$$TiRC = CiRC + CiRC \times FF/2,$$

in the above equation,

TiRC is the read count in the target chromosome i in a chromosome aneuploidy fetus,
CiRC is the read count in the target chromosome i in a normal fetus, and
FF is the fetal DNA fraction.

5. The method according to claim 1, wherein producing virtual positive data from normal data is determined by the following equation when the target chromosome is XO chromosome:

$$CXRC = CXXRC - CXXRC \times FF/2,$$

in the above equation,

CXRC is the read count in the target chromosome XO in a chromosome aneuploidy fetus,
CXXRC is the read count in the target chromosome XX in a normal female fetus, and
FF is the fetal DNA fraction.

6. The method according to claim 1, wherein producing virtual positive data from normal data is determined by the following equation when the target chromosome is XXX chromosome:

$$CXXXRC = CXXRC + CXXRC \times FF/2,$$

in the above equation,

CXXXRC is the read count in the target chromosome XXX in a chromosome aneuploidy fetus,
CXXRC is the read count in the target chromosome XX in a normal female fetus, and
FF is the fetal DNA fraction.

7. The method according to claim 1, wherein producing virtual positive data from normal data is determined by the following equation when the target chromosome is XYY chromosome:

$$CXYYRC = CXYRC + CYRC,$$

in the above equation,

CXYYRC is the read count in the target chromosome XYY in a chromosome aneuploidy fetus,
CXYRC is the read count in the target chromosome XY in a normal male fetus, and
CYRC is the read count assigned to Y chromosome in a normal male fetus.

8. The method according to claim 1, wherein producing virtual positive data from normal data is determined by the following equation when the target chromosome is XXY chromosome:

$$CXXYRC = CXXRC + CYRC,$$

in the above equation,

CXXYRC is the read count in the target chromosome XXY in a chromosome aneuploidy fetus,
CXXRC is the read count in the target chromosome XX in a normal female fetus, and
CYRC is the read count assigned to Y chromosome.

9. The method according to claim 8, wherein the CYRC is calculated by a method comprising:

calculating a residual from the following equations 1 and 2:

$$CYRC = \beta_0 + \beta_1 TRC + \beta_2 FF_{chrY} + \varepsilon \quad \text{(equation 1),}$$

in the above equation 1, TRC is the read count in the entire chromosome, $FF_{chrY}$ is FF based on the Y chromosome, $\beta_0$ is an intercept, $\beta_1$ is the coefficient between TRC and CYRC, $\beta_2$ is the coefficient between $FF_{chrY}$ and CYRC, and $\varepsilon$ is a residual,

$$CYRC' = \beta_0 + \beta_1 TRC + \beta_2 FF_{chrY} \quad \text{(equation 2),}$$

in the above equation 2, CYRC' is the predicted value of CYRC, TRC is the read count in the entire chromosome, $FF_{chrY}$ is FF based on the Y chromosome, $\beta_0$ is an intercept, $\beta_1$ is the coefficient between TRC and CYRC', and $\beta_2$ is the coefficient between $FF_{chrY}$ and CYRC'; and
calculating CYRC from the residual.

10. The method according to claim 1, wherein establishing a chromosome aneuploidy discrimination model from the produced virtual positive data and normal data is performed based on CiRC (chromosome i read count) (i = 1 to 22), ATRC (autosomal total read count), FF, and GC content when the target chromosome is an autosome.

11. The method according to claim 1, wherein detecting chromosome aneuploidy of a test sample using the chromosome aneuploidy discrimination model is performed based on CiRC (i = 1 to 22), ATRC, FF, and GC content when the target chromosome is an autosome.

12. The method according to claim 1, wherein establishing a chromosome aneuploidy discrimination model from the produced virtual positive data and normal data is performed based on CXRC (chromosome X read count), CYRC (chromosome Y read count), or a combination thereof; TRC (total read count); FF; and GC content when the target chromosome is a sex chromosome.

13. The method according to claim 1, wherein detecting chromosome aneuploidy of a test sample using the chromosome aneuploidy discrimination model is performed based on CXRC, CYRC, or a combination thereof; TRC, FF, and GC content when the target chromosome is a sex chromosome.

14. The method according to claim 1, wherein the chromosome aneuploidy discrimination model is trained by applying a machine learning algorithm.

15. The method according to claim 14, wherein the machine learning algorithm is selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis).

16. A computer-readable medium for recording a program applied to perform the method according to any one of claims 1 to 15.

Figure 1a

Figure 1b

Figure 1c

## T13 positive virtual data

### Learning data

|  | Negative | Positive |
|---|---|---|
| **Actual** Negative | 12865 | 1 |
| **Actual** Positive | 6 | 11749 |

Negative Positive
Prediction

Sensitivity = 99.95%

Positive predictive value = 99.99%

### Test data

|  | Negative | Positive |
|---|---|---|
| **Actual** Negative | 3126 | 7 |
| **Actual** Positive | 16 | 3007 |

Negative Positive
Prediction

Sensitivity = 99.47%

Positive predictive value = 99.77%

Figure 1d

# T13 actual data

|  | Negative | Positive |
|---|---|---|
| Negative | 200 | 0 |
| Positive | 0 | 6 |

Actual

Negative Positive

## Prediction

Sensitivity = **99.47%**

Positive predictive value = **99.77%**

Figure 2a

Figure 2b

Figure 2c

# T18 positive virtual data

## Learning data

|  | Negative | Positive |
|---|---|---|
| Negative | 12843 | 0 |
| Positive | 4 | 12581 |

Actual / Prediction

Sensitivity = 99.97%

Positive predictive value = 100%

## Test data

|  | Negative | Positive |
|---|---|---|
| Negative | 3147 | 6 |
| Positive | 16 | 3189 |

Actual / Prediction

Sensitivity = 99.50%

Positive predictive value = 99.81%

Figure 2d

# T18 Actual data

|  | 200 | 0 |
|---|---|---|
| Negative Actual | | |
| Positive | 0 | 12 |

Negative Positive

## Prediction

Sensitivity = 100%

Positive predictive value = 100%

Figure 3a

Figure 3b

Figure 3c

# T21 positive virtual data

## Learning data

|  | Negative | Positive |
|---|---|---|
| **Negative** | 12851 | 0 |
| **Positive** | 2 | 12245 |

Actual / Prediction

Sensitivity = 99.98%

Positive predictive value = 100%

## Test data

|  | Negative | Positive |
|---|---|---|
| **Negative** | 3131 | 2 |
| **Positive** | 3 | 3139 |

Actual / Prediction

Sensitivity = 99.90%

Positive predictive value = 99.94%

Figure 3d

**Actual data**

|  | Negative | Positive |
|---|---|---|
| **Negative** | 200 | 0 |
| **Positive** | 0 | 112 |

Actual

Negative Positive
Prediction

Sensitivity = 100%
Positive predictive = 100%
value

Figure 4a

Figure 4b

Figure 4c

# Sex chromosome positive virtual data

## Learning data

Actual / Prediction confusion matrix:

| Actual \ Prediction | XY | XX | XYY | XO | XXX | XXY |
|---|---|---|---|---|---|---|
| XY | 6244 | 0 | 1 | 1 | 0 | 5 |
| XX | 0 | 5911 | 0 | 13 | 22 | 0 |
| XYY | 0 | 0 | 6252 | 0 | 0 | 0 |
| XO | 0 | 12 | 0 | 5892 | 1 | 0 |
| XXX | 0 | 14 | 0 | 4 | 5933 | 0 |
| XXY | 2 | 0 | 0 | 0 | 0 | 5269 |

### Prediction

| Item | Sensitivity (%) | Positive predictive value(%) |
|---|---|---|
| XY | 99.89 | 99.97 |
| XX | 99.41 | 99.56 |
| XYY | 100 | 99.98 |
| XO | 99.78 | 99.70 |
| XXX | 99.70 | 99.61 |
| XXY | 99.96 | 99.91 |
| 전체 | 99.79 | 99.79 |

## Test data

| Actual \ Prediction | XY | XX | XYY | XO | XXX | XXY |
|---|---|---|---|---|---|---|
| XY | 1536 | 0 | 0 | 0 | 0 | 14 |
| XX | 0 | 1465 | 0 | 9 | 10 | 1 |
| XYY | 0 | 0 | 1549 | 0 | 0 | 0 |
| XO | 0 | 9 | 0 | 1515 | 1 | 1 |
| XXX | 0 | 12 | 0 | 1 | 1466 | 1 |
| XXY | 10 | 0 | 0 | 0 | 0 | 1294 |

### Prediction

| Item | Sensitivity (%) | Positive predictive value(%) |
|---|---|---|
| XY | 99.10 | 99.35 |
| XX | 98.65 | 98.59 |
| XYY | 100 | 100 |
| XO | 99.28 | 99.34 |
| XXX | 99.05 | 99.26 |
| XXY | 99.23 | 98.70 |
| 전체 | 99.22 | 99.21 |

Figure 4d

## Learning data

| Actual | | XY | XX | XYY | XO | XXX | XXY |
|---|---|---|---|---|---|---|---|
| | XY | 200 | 0 | 0 | 0 | 0 | 0 |
| | XX | 0 | 200 | 0 | 0 | 0 | 0 |
| | XYY | 0 | 0 | 2 | 0 | 0 | 0 |
| | XO | 0 | 0 | 0 | 7 | 0 | 0 |
| | XXX | 0 | 0 | 0 | 0 | 14 | 0 |
| | XXY | 0 | 0 | 0 | 0 | 0 | 8 |

## Prediction

| Item | Sensitivity (%) | Positive Predictive value (%) |
|---|---|---|
| XY | 100 | 100 |
| XX | 100 | 100 |
| XYY | 100 | 100 |
| XO | 100 | 100 |
| XXX | 100 | 100 |
| XXY | 100 | 100 |
| 전체 | 100 | 100 |

<table>
<tr><td colspan="2">**INTERNATIONAL SEARCH REPORT**</td><td>International application No.<br>**PCT/KR2022/017675**</td></tr>
</table>

**A.    CLASSIFICATION OF SUBJECT MATTER**

**G16B 20/00**(2019.01)i; **G16B 30/00**(2019.01)i; **G16B 40/00**(2019.01)i

According to International Patent Classification (IPC) or to both national classification and IPC

**B.    FIELDS SEARCHED**

Minimum documentation searched (classification system followed by classification symbols)

G16B 20/00(2019.01); C12Q 1/68(2006.01); G16B 10/00(2019.01); G16B 20/20(2019.01); G16B 30/00(2019.01); G16B 40/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 태아 염색체이수성 검출(fetal aneuploidy detection), 리드 맵핑(read mapping), 리드수(read count), 태아 DNA 분획(fetal DNA fraction), GC 함량(GC contents), 가상의 염색체이수성 데이터(virtual aneuploidy data), 판별 모델(distinguishing model)

**C.    DOCUMENTS CONSIDERED TO BE RELEVANT**

| Category* | Citation of document, with indication, where appropriate, of the relevant passages | Relevant to claim No. |
|---|---|---|
| A | KR 10-2021-0067931 A (GREEN CROSS GENOME CORPORATION) 08 June 2021 (2021-06-08)<br>      See paragraphs [0007]-[0008], [0027] and [0087]; claims 1, 9 and 12; and figure 4. | 1-16 |
| A | US 2018-0073080 A1 (NATERA, INC.) 15 March 2018 (2018-03-15)<br>      See abstract; paragraph [0015]; and claim 5. | 1-16 |
| A | KR 10-2017-0088780 A (GENOMECARE CO., LTD.) 02 August 2017 (2017-08-02)<br>      See entire document. | 1-16 |
| A | KR 10-2016-0013183 A (SEQUENOM, INC.) 03 February 2016 (2016-02-03)<br>      See entire document. | 1-16 |
| A | YANG, Jianfeng et al. Improving the calling of non-invasive prenatal testing on 13-/18-/21-trisomy by support vector machine discrimination. PLoS ONE. 2018, vol. 13, no. 12, article no. e0207840, pp. 1-19.<br>      See entire document. | 1-16 |

☐ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 March 2023** | **02 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2022/017675** |

| Patent document cited in search report | | | Publication date (day/month/year) | Patent family member(s) | | | Publication date (day/month/year) |
| --- | --- | --- | --- | --- | --- | --- | --- |
| KR | 10-2021-0067931 | A | 08 June 2021 | CA | 3163405 | A1 | 03 June 2021 |
| | | | | EP | 4068291 | A1 | 05 October 2022 |
| | | | | JP | 2023-504139 | A | 01 February 2023 |
| | | | | US | 2023-0028790 | A1 | 26 January 2023 |
| | | | | WO | 2021-107676 | A1 | 03 June 2021 |
| US | 2018-0073080 | A1 | 15 March 2018 | BR | 112013016193 | A2 | 19 June 2018 |
| | | | | CN | 102449208 | A | 09 May 2012 |
| | | | | CN | 102449208 | B | 10 December 2014 |
| | | | | EP | 2455515 | A1 | 23 May 2012 |
| | | | | EP | 2455515 | A4 | 27 March 2013 |
| | | | | EP | 2455515 | B1 | 10 January 2018 |
| | | | | JP | 2014-502845 | A | 06 February 2014 |
| | | | | JP | 5234184 | B2 | 10 July 2013 |
| | | | | KR | 10-1890466 | B1 | 21 August 2018 |
| | | | | KR | 10-2015-0038216 | A | 08 April 2015 |
| | | | | US | 11306357 | B2 | 19 April 2022 |
| | | | | US | 2011-0288780 | A1 | 24 November 2011 |
| | | | | US | 2022-0042103 | A1 | 10 February 2022 |
| | | | | US | 8825412 | B2 | 02 September 2014 |
| | | | | WO | 2011-007457 | A1 | 20 January 2011 |
| KR | 10-2017-0088780 | A | 02 August 2017 | KR | 10-1739535 | B1 | 24 May 2017 |
| | | | | KR | 10-1881098 | B1 | 24 July 2018 |
| | | | | US | 2019-0103187 | A1 | 04 April 2019 |
| | | | | WO | 2017-131359 | A1 | 03 August 2017 |
| KR | 10-2016-0013183 | A | 03 February 2016 | CN | 105555968 | A | 04 May 2016 |
| | | | | CN | 105555968 | B | 23 October 2020 |
| | | | | EP | 3004383 | A2 | 13 April 2016 |
| | | | | EP | 3004383 | B1 | 24 April 2019 |
| | | | | HK | 1217033 | A1 | 16 December 2016 |
| | | | | JP | 2016-526879 | A | 08 September 2016 |
| | | | | JP | 6561046 | B2 | 14 August 2019 |
| | | | | KR | 10-2022-0048042 | A | 19 April 2022 |
| | | | | KR | 10-2385062 | B1 | 12 April 2022 |
| | | | | US | 10699800 | B2 | 30 June 2020 |
| | | | | US | 2016-0224724 | A1 | 04 August 2016 |
| | | | | WO | 2014-190286 | A2 | 27 November 2014 |
| | | | | WO | 2014-190286 | A3 | 26 February 2015 |

Form PCT/ISA/210 (patent family annex) (July 2022)

**EP 4 439 565 A1**

## REFERENCES CITED IN THE DESCRIPTION

**Patent documents cited in the description**

- KR 1739535 B

**Non-patent literature cited in the description**

- **GUOLIN KE et al.** LightGBM: A Highly Efficient Gradient Boosting Decision Tree. *Proceedings of the 31st International Conference on Neural Information Processing Systems,* 2017, 3149-3157